# EUROPEAN PATENT SPECIFICATION

(11) **EP 0 829 515 B1**
(45) Date of publication and mention of the grant of the patent: **27.03.2002**
(21) Application number: 97114020.7
(22) Date of filing: 31.07.1992
(51) Int. Cl.: C08L 35/08, C08K 5/00, A61C 13/23

(54) **Stabilized aqueous solution**
Stabilisierte wässrige Lösung
Solution aqueuse stabilisée

(30) Priority: 17.09.1991 US 761089; 18.09.1991 US 761535
(43) Date of publication of application: 18.03.1998
(62) Divisional of application: 92916901.9
(73) Proprietor: ISP INVESTMENTS INC., Wilmington, Delaware 19801 (US)
(72) Inventor: Plochocka, Krystyna, Scotch Plains, NJ 07076 (US); Chuang, Jui-Chang, Wayne, NJ 07470 (US)
(74) Representative: Harrison, David Christopher

(56) References cited:
- GB-A- 906 230
- US-A- 3 531 427

## Description

This invention relates to the stabilization of aqueous solutions of polymers.

US-A-3 531427 discloses stabilization of polybasic acid/alkyl vinyl ehter copolymer solutions by use of a mixture of EDTA and a second component.

What is provided herein is a colorless, stabilized aqueous solution of a C₁-C₅ alkyl vinyl ether and maleic acid copolymer which includes 100 to 1,000 ppm of an additive mixture of (a) 30-70% by weight of ethylenediaminetetraacetic acid and (b) 30-70% by weight of a secondary additive which is organic antioxidant t-butylhydroquinone, said stabilized solution exhibiting a retention of at least about 90% of its original viscosity after 3 months.

Preferably, the stabilized solution is prepared by hydrolyzing the corresponding anhydride in the presence of said additive mixture.

In one embodiment, copolymers of maleic anhydride and a C₁-C₅ alkyl vinyl ether are dissolved in water containing 100-1,000 ppm of an additive mixture of (a) 30-70% by weight of ethylenediaminetetraacetic acid and (b) 30-70% by weight of a secondary additive which is organic antioxidant t-butylhydroquinone. The polymer reaction solution containing the predetermined additive then is hydrolyzed to the corresponding acid form of the polymer to provide the desired colorless, stabilized aqueous solution of maleic acid-C₁-C₅ alkyl vinyl ether copolymer which includes the additive mixture.

While the additive mixture could be added after hydrolysis, it is much preferred to add it directly to the anhydride copolymer before hydrolysis. In this manner, solution relative viscosities of 11-14 measured 1% in water at 25°C., are obtained for a typical high molecular weight maleic acid-C₁-C₅-alkyl vinyl ether copolymer instead of the value of 9 when untreated and 9-11 when the additive is included in the solution after hydrolysis.

The secondary additive of the additive mixture of the invention has the following particular attributes:
(1) effective in stabilizing the viscosity of the interpolymer;
(2) useful in small amounts;
(3) soluble in water;
(4) does not discolor the solution;
(5) an effective antioxidant and/or free radical scavenger; and
(6) t-butylhydroquinone is suitable for use in denture adhesives and other personal care products.

### COMPARATIVE EXAMPLES

### 1. Untreated Copolymer

A 1-liter resin kettle equipped with an agitator, a reflux condenser and a water bath was charged with 22.0 g. of methyl vinyl ether/maleic anhydride copolymer (Gantrez ® AN-169, International Specialty Products, Inc.) of specific viscosity of 2.83 (as determined in methyl ethyl ketone at a concentration of 1% at 25°C.) and 178.0 g. of deionized water. While agitating, the mixture was heated to 85°C. and maintained at this temperature for 1.5 hours. During this period the anhydride groups of the copolymer were converted to carboxylic acid groups generating a methyl vinyl ether/maleic acid copolymer. The clear solution which contained 13.02% solids was cooled to room temperature and maintained, in the dark, for 3 months. The stability of the solution was determined by measuring its specific viscosity (1% in H₂O, at 25°C.) and Brookfield viscosity (Spindle # 3, 5 rpm, 25°C.). The results were the following:

**TABLE 1**

| Time, months | Spec. Viscosity | Brookfield Viscosity, cps |
|---|---|---|
| 0 | 9.68 | 2160 |
| 3 | 5.84 | 600 |

The data shows that a solution of methyl vinyl ether/maleic acid copolymer, which did not contain any stabilizer, deteriorated to only 40% of its initial specific viscosity, and to about 28% of its initial Brookfield viscosity, after a period of only 3 months.

### 2. Individual Additives

To the solution prepared in Example 1, a number of different additives were added after dissolution of the polymer. The following data show the stabilizing effect of these additives:

**TABLE 2**

| Additive/ppm | Time, months | Spec. Viscosity | Brookfield Viscosity, cps |
|---|---|---|---|
| None | 0 | 9.68 | 2160 |
| | 3 | 5.84 | 600 |
| Ethylenediaminetetraacetic acid (EDTA)/200 ppm | 0 | 9.68 | 2160 |
| | 3 | 8.65 | 2050 |
| t-Butyl hydroquinone (TBHQ)/500 ppm | 0 | 9.68 | 2160 |
| | 3 | 8.90 | 1940 |
| N,N-Diethylhydroxylamine (DEHA)/500 ppm | 0 | 9.68 | 2160 |
| | 3 | 8.78 | 2000 |
| Propyl gallate (PG)/500 | 0 | 9.68 | 2160 |
| | 3 | 8.70 | 1900 |

The data above shows that the addition of a stabilizing amount of either a transition metal chelating agent, (EDTA), or of an antioxidant, (TBHQ, PG), or a radical chaser, (DEHA), substantially increased the stability of the acidic copolymer; and, particularly, it degraded to a significantly lesser degree than a nonstabilized copolymer solution of Comparative Example 1.

### INVENTION EXAMPLES

### 1. Additive Mixture After Hydrolysis

To the solution of Example 1 various stabilizer blends were added. The stabilizing effect is apparent in the data in Table 3 below.

**TABLE 3**

| Additive Blend/ppm | Time, months | Spec. Viscosity | Brookfield Viscosity, cps |
|---|---|---|---|
| None | 0 | 9.68 | 2160 |
| | 3 | 5.84 | 600 |
| TBHQ/500 + EDTA/200 | 0 | 9.68 | 2160 |
| | 3 | 9.00 | 2200 |

The data shows an improvement over the individual components of Comparative Example 2. Specifically, the specific viscosity of the copolymer decreased by only 7.6% and the Brookfield viscosity of the copolymer solution was substantially unchanged after standing for 3 months at room temperature.

### 2. Additive Mixture Before Hydrolysis

A copolymer solution as in Example 1 was prepared by dissolving Gantrez® AN-169 in an additive blend of various stabilizers and water prior to beginning of heating. The acid copolymer was made by heating as in Example 1. The results are shown in Table 4 below.

**TABLE 4**

| Additive Blend/ppm | Time, months | Spec. Viscosity | Brookfield Viscosity, cps |
|---|---|---|---|
| TBHQ/75+ | 0 | 11.75 | 2800 |
| EDTA/75 | 3 | 10.87 | 2900 |

The data above demonstrates the advantage of adding the stabilizers during dissolution of copolymer. Accordingly, both the specific viscosity and the Brookfield viscosity values of the solution were at least 90% of its initial values after 3 months, and with much higher initial and final viscosities with much lower concentrations of the mixture, than the same additive mixture used after hydrolysis.

## Claims

1. A colorless, stabilized aqueous solution of a copolymer of a C₁-C₅ alkyl vinyl ether and maleic acid suitable for use in denture adhesives which includes 100 to 1,000 ppm of an additive mixture of (a) 30-70% by weight of ethylenediaminetetraacetic acid and (b) 30-70% by weight of a secondary additive which is the organic antioxidant t-butylhydroquinone, said stabilized solution exhibiting a retention of at least 90% of its original viscosity after a period of 3 months.

2. A solution according to claim 1 wherein said solution is prepared by hydrolyzing a copolymer of a C₁-C₅ alkyl vinyl ether and maleic anhydride in the presence of said additive mixture.

3. A solution according to claim 2 which includes about 500 ppm of said additive mixture.

4. A solution according to claim 1 which includes about 50% each of (a) and (b).

## Patentansprüche

1. Farblose, stabilisierte wässrige Lösung eines Copolymers einea C₁-C₅-Alkylvinylethers mit Maleinsäure, die sich zur Verwendung für Gebisshaftmittel eignet und Folgendes umfasst: 100 bis 1.000 ppm eines Additivgemisches aus (a) 30 bis 70 Gew.-% Ethylendiamintetraessigsäure und (b) 30 bis 70 Gew.-% eines sekundären Additivs, das der organische Antioxidans t-Butylhydrochinon ist, wobei die stabilisierte Lösung während eines Zeitraums von 3 Monaten zumindest 90 % ihrer ursprüngliche Viskosität beibehält.

2. Lösung nach Anspruch 1, worin die Lösung durch Hydrolysieren eines Copolymers eines C₁-C₅-Alkylvinylethers mit Maleinsäureanhydrid in Gegenwart des Additivgemisches hergestellt wird.

3. Lösung nach Anspruch 2, die etwa 500 ppm des Additivgemisches enthält.

4. Lösung nach Anspruch 1, die jeweils etwa 50 % an (a) und (b) enthält.

## Revendications

1. Solution aqueuse incolore, stabilisée, d'un copolymère d'éther d'alkyle en C₁-C₅ vinyle et d'acide maléique appropriée pour une utilisation dans des adhésifs pour dentier qui comprend 100 à 1000 ppm d'un mélange d'additifs de (a) 30-70% en poids d'acide éthylènediaminetétraacétique et de (b) 30-70% en poids d'un deuxième additif qui est l'antioxydant organique t-butylhydroquinone, ladite solution stabilisée présentant une rétention d'au moins 90% de sa viscosité d'origine après une période de 3 mois.

2. Solution suivant la revendication 1, dans laquelle ladite solution est préparée par hydrolyse d'un copolymère d'éther d'alkyle en C₁-C₅ vinyle et d'anhydride maléique en présence dudit mélange d'additifs.

3. Solution suivant la revendication 2, qui comprend environ 500 ppm dudit mélange d'additifs.

4. Solution suivant la revendication 1, qui comprend environ 50% de chacun de (a) et (b).
